Europäisches Patentamt

European Patent Office

Office européen des brevets

(19)

(11) Numéro de publication : **0 287 480 B1**

(12)

# FASCICULE DE BREVET EUROPEEN

(45) Date de publication du fascicule du brevet :
**11.12.91 Bulletin 91/50**

(51) Int. Cl.[5] : **B65B 55/02, A23L 3/26, A61L 2/02**

(21) Numéro de dépôt : **88420109.6**

(22) Date de dépôt : **01.04.88**

(54) **Procédé et dispositif pour rendre stérile le contenu de récipients en matériau diélectrique.**

(30) Priorité : **10.04.87 FR 8705461**

(43) Date de publication de la demande :
**19.10.88 Bulletin 88/42**

(45) Mention de la délivrance du brevet :
**11.12.91 Bulletin 91/50**

(84) Etats contractants désignés :
**AT BE CH DE ES FR GB GR IT LI LU NL SE**

(56) Documents cités :
**FR-A- 745 354**
**FR-A- 823 050**
**FR-A- 2 127 406**
**US-A- 3 753 886**
**US-A- 4 309 388**

(73) Titulaire : **Georjon, Jean**
**La Faverge**
**F-42320 La Grand Croix (FR)**
Titulaire : **Granger, Gaston**
**Le Calvaire**
**F-43000 Sainte Sigolene Haute-Loire (FR)**

(72) Inventeur : **Georjon, Jean**
**La Faverge**
**F-42320 La Grand Croix (FR)**

(74) Mandataire : **Perrier, Jean-Pierre et al**
**Cabinet GERMAIN & MAUREAU 12 rue de la République**
**F-42000 St-Etienne (FR)**

EP 0 287 480 B1

## Description

La stérilisation des produits ou fluides disposés dans un contenant, étanche,aux bactéries, est de plus en plus soumise à des contraintes de rapidité éliminant les procédés traditionnels de pasteurisation, en général longs, au profit de procédés mettant en oeuvre des champs électromagnétiques ou des rayonnements divers, plus rapides, mais exigeant un investissement important et entrainant des contraintes supplémentaires pour assurer la sécurité du personnel.

La présente invention est relative à l'application d'un autre procédé de stérilisation consistant à exposer, temporairement, contenant et contenu, à des effluves électriques générées, par effet CORONA, par des électrodes alimentées par un courant électrique alternatif à haute tension et basse fréquence.

Le brevet FR-A-745354 décrit des dispositifs dans lesquels des électrodes rotatives porteuses de pointes de décharge et alimentées par un courant de 170000 volts avec une fréquence de l'ordre de 680 hertz, sont disposées à distance d'une contre électrode, et forment avec cette dernière un espace de traitement dans lequel sont disposés, d'une part, un tapis isolant pour le transport des produits à stériliser et, d'autre part, une plaque isolante supportant le tapis. Cette dernière est réalisée dans un matériau ayant une grande impédance et comporte, en dessous des électrodes rotatives, des ouvertures modifiant localement l'impédance pour concentrer le flux de décharge partant des pointes des électrodes rotatives et pour l'obliger à traverser le produit dans cette zone du tapis transporteur.

En pratique, une telle installation mettant en oeuvre une tension élevée à basse fréquence et générant des décharges sur des distances de l'ordre de 90 cm, est dangereuse pour le personnel qui peut être électrocuté par les décharges,mais aussi par les courants de fuites, malgrè l'existence de plaques métalliques supplémentaires de concentration du flux.

Par ailleurs, l'émission des décharges, plus communément appelées effluves, à partir de pointes de décharge, même tournant à vitesse élevée, pour hacher ces décharges, peut, malgrè les nombreuses précautions prises, favoriser la formation d'arcs électriques aptes à détruire les récipients et éventuellement leur contenu.

Cette installation fournit des effluves se déplaçant suivant une trajectoire circulaire, et formant un flux cylindrique ayant une section en forme de couronne offrant une faible surface de traitement et conduisant à un temps de traitement long, incompatible avec les cadences de fabrication actuelle. Enfin, une telle installation conçue pour une action stérilisatrice, consistant à tuer des insectes ou leurs oeufs, ne peut pas être utilisé pour rendre stérile le contenu de récipients en matière synthétique étanches, d'autant plus que ceux-ci risqueraient d'être perforés par le fort courant de décharge.

La présente invention a donc pour but de fournir un procédé et un dispositif pour rendre stérile le contenu de récipients en matériau diélectrique, et en particulier, quoique que non exclusivement,en matière synthétique, dispositif qui soit sans danger pour le personnel, procure des effluves régulières réparties suivant une surface de traitement importante, soit peu onéreux et puisse assurer une stérilisation totale et réglable du contenu, dans un temps compatible avec les cadences de fabrication actuelle.

Ce procédé est du type consistant à disposer les récipients entre des électrodes reliées à un générateur de courant électrique alternatif.

Selon l'invention, chaque récipient est maintenu au contact de l'une des électrodes pendant la stérilisation, et une meilleure répartition des effluves est obtenue en masquant la partie métallique d'au moins l'une de ces électrodes par une couche en matériau diélectrique ayant une surface lisse sans aspérités et avec un état de surface poli, tandis que ces électrodes sont alimentées par un courant électrique alternatif de tension comprise entre 10000 et 50000 Volts avec une fréquence d'au moins 3000 hertz.

Dans le dispositif pour la mise en oeuvre de ce procédé, au moins l'une des électrodes est composée d'une partie conductrice à laquelle est associée, sur sa face faisant vis-à-vis à l'autre électrode, une couche en matériau isolant constituant diélectrique d'égalisation et de répartition des effluves, tandis que, d'une part, au moins l'une de ces électrodes est en contact avec le récipient, que d'autre part toutes les électrodes présentent des surfaces lisses, sans aspérités, avec un état de surface poli, et dont au moins les génératrices sont parallèles à celles de l'autre électrode, et que, de plus,les électrodes en vis-à-vis sont séparées par un intervalle au plus égal à la hauteur du récipient, augmenté d'une valeur comprise entre 0 et 5 mm, et sont reliées à un générateur apte à délivrer un courant alternatif de tension réglable comprise entre 10000 et 50000 volts et une fréquence d'au moins 3000 Hertz.

Grâce à l'état de surface poli des électrodes et à leur parallélisme, ce dispositif génère un flux d'effluves d'autant plus régulier que celles-ci sont réparties par le couche de matériau diélectrique accolé à l'une des électrodes. Il en résulte que, la formation d'arcs électriques est considérablement atténuée. En outre, les caractéristiques du courant d'alimentation sont celles des courants qui, dits superficiels, circulent en surface sur l'homme et sont sans danger cardiaque pour lui.

Dans une forme d'exécution concernant le traitement de récipients étanches comprenant un produit ou fluide dont il faut assurer la stérilisation, ces récipients étant véhiculés par une bande transporteuse, l'une des électrodes est constituée par la bande sans fin, réalisée en matériau conducteur de l'électricité, tandis que l'autre électrode est constituée par une plaque métallique qui, disposée au dessus de la bande transporteuse est solidaire d'une couche en matériau isolant faisant vis-à-vis à cette bande.

Lors du passage du récipient isolant entre les électrodes sous tension, le courant ne circule pas sur le récipient, mais traverse celui-ci en formant, dans la partie exposée, composée d'une couche d'air et du fluide ou produit contenu dans le récipient, des effluves qui sont visibles jusqu'à transformation en ozone de l'oxygène de la couche d'air. Il en résulte que les bactéries situées dans le champ électrique sont détruites et que celles qui, dans le contenu n'auraient pas été détruites, le sont par la suite par l'action stérilisatrice de l'ozone formée dans le récipient.

Dans une autre forme d'exécution, visant à rendre stérile des récipients formés par des nappes en matériau diélectrique superposée, l'une des électrodes est constituée par un cylindre qui, d'axe transversal à la direction de déplacement des récipients en nappes, est composé d'un corps tubulaire en matériau diélectrique recouvert intérieurement par une couche en matériau conducteur relié électriquement au générateur, tandis que l'autre électrode est constituée par une plaque métallique en forme de gouttière coiffant sans le toucher le cylindre précité.

Avec cet agencement, après leur fabrication, les récipients sont stérilisés en continu. Après traitement, leur cavité interne reste stérile jusqu'à leur remplissage par des produits stériles ou non quine risquent donc pas d'être pollués par le récipient.

Enfin, dans une autre forme d'exécution concernant la stérilisation d'un fluide apte à circuler dans un récipient raccordé aux tronçons d'arrivée et de départ du fluide, le récipient est constitué par un caisson en matériau diélectrique et étanche,ayant une hauteur intérieur supérieur au diamètre intérieur des tronçons et délimitant, entre sa paroi surpérieure et le niveau supérieur du fluide qu'il contient, une chambre de traitement contenant de l'air, tandis que, d'une part, les électrodes opposées sont disposées à l'extérieur du caisson, respectivement au dessus de sa face externe supérieure et au dessous de sa face externe inférieure et que, d'autre part, débouchent dans la chambre supérieure de traitement des canalisations, respectivement, d'amenée et d'évacuation d'air sous pression associées des moyens régulant le débit d'air sous pression pour maintenir un niveau de fluide sensiblement constant dans le récipient.

En fonctionnement, les effluves se forment dans la couche d'air de la chambre de traitement et traversent le fluide, qui constitue alors conducteur de ces effluves, pour rejoindre l'autre électrode, en réalisant ainsi la stérilisation de l'air et du fluide et en tranformant en ozone l'oxygène de la couche d'air, mais aussi de l'air contenu dans les bulles en suspension dans l'eau.

D'autres caractéristiques et avantages ressortiront de la description qui suit en référence au dessin schématique annexé représentant, à titre d'exemples non limitatifs, des formes d'exécution du dispositif dans diverses applications.

Figure 1 est une vue générale de côté en élévation d'une forme d'exécution du dispositif, dans le cas de son application à la stérilisation du contenu de récipients étanches ;

Figures 2 et 3 sont des vues, respectivement, de côté et de face en élévation montrant l'application du dispositif de figure 1 à la stérilisation du contenu de récipients prismatiques ;

Figure 4 est une vue similaire à la figure 3 montrant le dispositif pour la stérilisation de récipients cylindriques ;

Figure 5 est une vue similaire à la figure 3 montrant l'application du dispositif à la stérilisation de bocaux fermés par des capsules métalliques ;

Figure 6 est une vue de côté, en coupe longitudinale,montrant le dispositif lorsqu'il est utilisé pour la stérilisation de produits alimentaires, ou autres, conditionnés dans un emballage formé par deux nappes en matière synthétique ;

Figure 7 est une vue de côté en coupe transversale d'une autre forme d'exécution du dispositif dans le cas de son application à la stérilisation d'un fluide contenu dans un récipient ;

Figure 8 est une vue de côté avec coupe d'une autre forme d'exécution du dispositif de figure 7 ;

Figure 9 est une vue partielle de côté en coupe d'une forme d'exécution du dispositif pour la stérilisation de récipients formés dans deux nappes en bande.

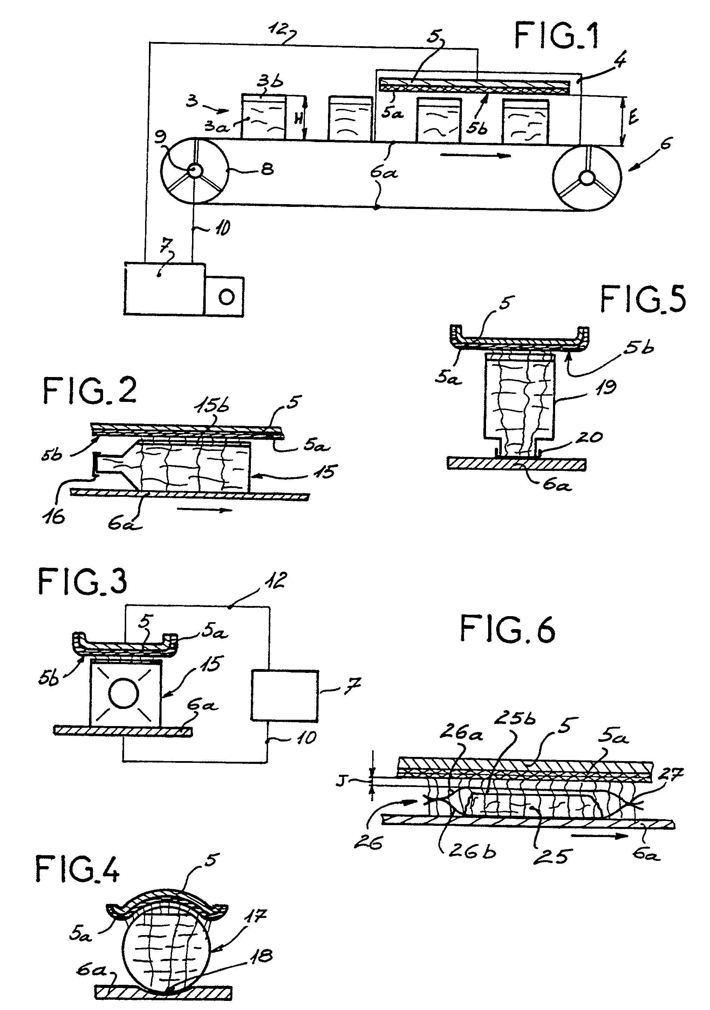

Dans son application à la stérilisation du contenu de récipients étanches, représenté aux figures 1 à 6, le dispositif selon l'invention, comprend un tapis transporteur 2, destiné à déplacer des récipients 3 pour les amener dans une enceinte de traitement 4, deux électrodes 5 et 6 reliées à un générateur 7 apte à délivrer un courant de tension réglable entre 10000 et 50000 volts a une fréquence d'au moins 3000 Hertz.

L'électrode inférieure est constituée par la bande 6a du tapis transporteur 6 qui est réalisée en matériau conducteur de l'électricité, et par exemple en métal. Cette bande passe sur une poulie de renvoi 8 dont l'arbre 9 est relié par un conducteur 10 à l'une des bornes du générateur 7.

L'électrode supérieure est constituée par une plaque métallique 5 en matériau conducteur de l'électricité, tel qu'en acier inoxydable ou en aluminium. Elle est reliée par un conducteur 12 à l'autre borne du générateur 7. Sur sa face, faisant vis-à-vis à la bande 6a, elle est recouverte par une couche 5a en matériau diélectrique d'égalisation et de répartition des effluves. La face 5b de cette couche est parallèle à la bande 6a. Elle présente une surface régulière, sans aspérités, ayant un état de surface de qualité polie, c'est-à-dire ne dépassant une rugosité R.A. de 0,4 selon les normes ISO/TC 57 et ASA B46-1955.

Les électrodes sont séparées par une distance E qui est au moins égale à la hauteur H des récipients et au plus à la valeur de cette hauteur augmentée d'une valeur de l'ordre de 5 mm, pour tenir compte des variations dimensionnelles de ces récipients.

Les récipients 3 sont réalisés en matériau diélectrique et étanche aux bactéries. Ils contiennent, d'une part, un produit, un liquide ou un fluide 3a, conducteur de l'électricité ou ayant une impédance inférieure à l'air entre les électrodes, et, d'autre part, une couche d'air 3b interposée entre le contenu et le moyen d'obturation du récipient.

Lorsque ces récipients sont amenés par la bande 6a dans l'enceinte de traitement 4, ils sont chacun soumis à un champ électrique alternatif se caractérisant par des effluves reliant les électrodes en traversant le récipient et son contenu, à savoir la couche d'air et le contenu réel. Les effluves créées dans la pellicule d'air 3b transforment l'oxygène en ozone. En traversant le produit 3a contenu dans le récipient, elles assurent la stérilisation de ce contenu. Il en résulte qu'à la fin du traitement, non seulement le contenu est stérilisé, mais qu'la couche d'air 3b est remplacée par un gaz dont les propriétés bactéricides garantissent la conservation dans le temps de cette stérilisation et rendent stérile le contenu du récipient.

La qualité de la stérilisation dépend de la tension d'alimentation des électrodes et du temps de passage de chaque récipient entre celles-ci, c'est-à-dire de deux paramètres pouvant être aisément réglés en intervenant respectivement sur les moyens de contrôle du générateur 7 et sur la vitesse de rotation du moteur entraînant le tapis 6 ou sur le temps d'arrêt des récipients au dessous de l'électrode 5.

Ce dispositif peut être utilisé pour traiter des produits liquides, ou fluides conducteurs, mais aussi pour traiter des produits non conducteurs. Dans le premier cas, et grâce à la conductibilité électrique du contenu, qui forme conducteur des effluves, le récipient peut avoir une grande hauteur sans perturber le traitement, pourvu que l'électrode supérieure soit en contact ou à faible distance de la face supérieure de ce récipient. Dans le second cas, la hauteur du récipient est limitée à une valeur variable, déterminée par l'impédance du récipient et de son contenu, impédance quine doit pas être supérieure ou égale à l'impédance de l'air entre les deux électrodes.

Il faut préciser, que grâce, d'une part, au parallélisme de l'électrode 5 et de la bande 6a, d'autre part, à l'état de surface de la face active 5b de cette électrode, et de plus, à la couche isolante 5a de répartition, les effluves sont régulièrement réparties, sur toute la surface de l'électrode, ont une très grande densité bien supérieure à celle obtenue par les électrodes de l'Art antérieur et ne risquent pas de se transformer en arcs électriques destructeurs.

Les figures 2 et 3 montrent que pour stériliser le contenu de récipients 15 en forme de bouteille de section transversale prismatique et notamment carrée, ces récipients sont couchés sur le tapis 6a du convoyeur. Cette disposition, favorable à la stabilité du récipient en déplacement, évite également tout amorçage d'arc entre les moyens de fermeture 16 du récipient et l'une ou l'autre des électrodes 5 ou 6. Lorsque le récipient est couché, l'air contenu dans celui-ci vient en partie supérieure, comme montré en 15b à la figure 2 et reste donc à proximité immédiate de l'électrode 5.

La figure 4 montre que, pour un récipient cylindrique 17, l'électrode supérieure 5 et sa couche isolante 5a sont cintrées en forme de tuile tandis que le tapis transporteur 6a comporte une gorge de positionnement 18.

La figure 5 montre la stérilisation de produits en pots 19, obturés par un bouchon conducteur de l'électricité 20. Pour éviter tout amorçage d'arc entre le bouchon 20 et l'électrode 5 par rapport à laquelle il se déplace, amorçage favorisé par les saillies du bouchon ou par les salissures portées par ce dernier, le récipient 19 est retourné de manière que le bouchon 20 repose sur la bande 6a et que sa base soit en vis à vis de l'électrode 5.

La figure 6 montre l'application du dispositif de figure 1 à la stérilisation de produits, fluides ou liquides 25, disposés, avec une couche d'air 25b, dans récipients 26 composés de deux nappes étanches 26a-26b, en matériau diélectrique, étanche aux bactéries, synthétique ou autre, et fermées, par des soudures transversales 27 et éventuellement longitudinales non représentées, ou par tous autres moyens. Le produit 25 peut être un produit alimentaire, tel que de la viande, ou un plat cuisiné ; ce peut être un vêtement, un pansement ou tout autre produit consommable ou non, conducteur de l'électricité ou non, et de manière générale, tout produit dont la conservation doit s'effectuer dans un milieu stérile ou qui doit être stérile avant d'être utilisé.

La couche 5a de l'électrode 5 peut être en contact avec le récipient 26 ou sans contact avec lui, la distance

EP 0 287 480 B1

J variant entre 0 et 5 mm.

Il ressort déjà que le dispositif selon l'invention peut être utilisé pour stériliser le contenu de tout récipient réalisé dans un matériau diélectrique, et ainsi être appliqué à la stérilisation d'eau en bouteille, de confitures, de pâtés, de plats cuisinés, de tranches de viande, de jambon et de tous autres produits pour la consommation, humaine ou animale, ou à usage médical conditionnés dans des récipients étanches aux bactéries.

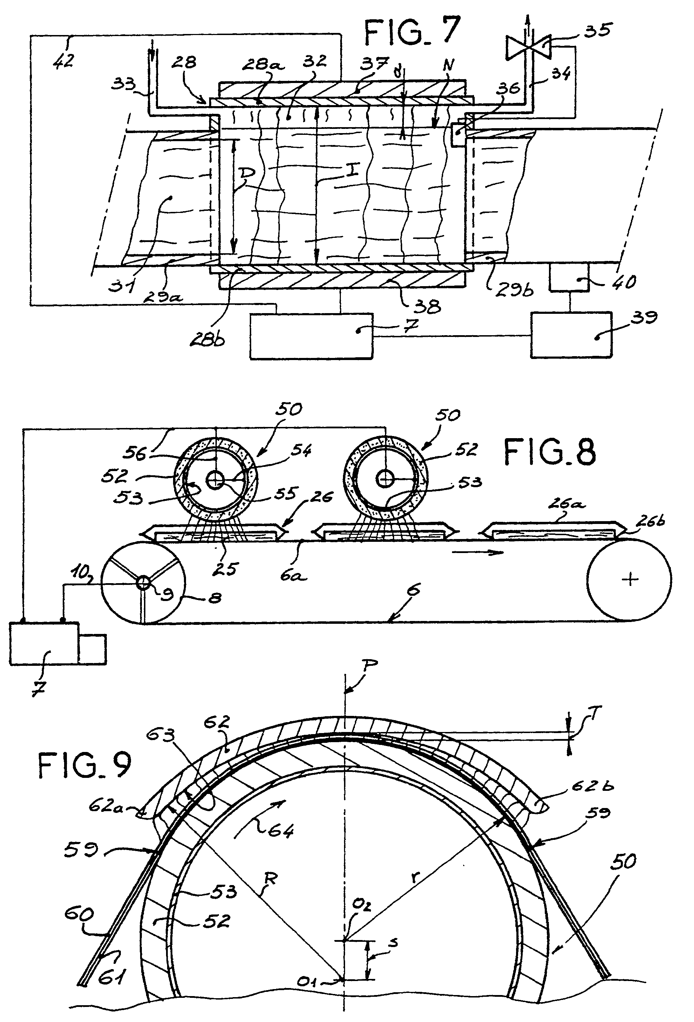

La figure 7 représente une forme d'exécution du dispositif pour la stérilisation d'un fluide 31, véhiculé par une conduite traversant un récipient et ayant une impédance inférieure à l'air.Le récipient est constitué par un caisson 28 en matériau diélectrique et étanche, tel qu'en verre, quartz, granit, porcelaine.Il est interposé entre les deux tronçons 29a-29b de la conduite. De section transversale rectangulaire, ce caisson a une hauteur intérieure I supérieure au diamètre intérieur D des tronçons 29a-29b de manière à délimiter une chambre de traitement 32 entre le niveau supérieur N du fluide et sa paroi supérieure 28a.

Cette chambre de traitement communique avec une canalisation 33 d'arrivée d'air sous pression et avec une canalisation de sortie d'air 34 munie d'un robinet 35. Le fonctionnement de ce robinet 35 est sous la dépendance d'un détecteur 36 du niveau de fluide dans le caisson 28.

La pression d'arrivée d'air est toujours supérieure, d'au moins un bar, à la pression du fluide dans le caisson 28. Le maintien de la hauteur a de la chambre 32 a une valeur de l'ordre de quelques millimètres, malgré les variations de débit ou de pression dans la conduite, est assuré par l'ouverture ou la fermeture du robinet 35. Ainsi par exemple, si la pression d'air dans la chambre 32 diminue, ce qui entraine une élévation du niveau N dans le caisson 28, le détecteur 36 commande la fermeture du robinet 35. Il en résulte que la pression dans la chambre 32 revient progressivement à la valeur de la pression d'admission de l'air et provoque un abaissement du niveau N sans pour autant qu'il parvienne au niveau maximal du fluide contenu dans les tronçons 29a-29b. A l'inverse, si ce niveau N s'abaisse trop et risque de venir au niveau maximal des tronçons précités, le détecteur 36 commande l'ouverture du robinet 35 pour créer une fuite abaissant la pression dans la chambre 32 et permettant l'élévation du niveau de fluide.

Cet agencement permet donc d'ajuster le niveau N à une valeur déterminée pour former, entre ce niveau et la paroi supérieure 28a du récipient 28 une couche d'air, d'épaisseur a, suffisante pour obtenir un bon effluvage.

Ce dispositif comprend également deux électrodes 37 et 38 disposées à l'extérieur du caisson, respectivement, au dessus de sa paroi supérieure 28a et au dessous de celle inférieure 28b. En pratique, les deux électrodes sont plaquées contre les faces externes des parois qui réalisées en matériau diélectrique et présentant des face internes parallèles avec un état de surface poli assurent la répartition des effluves. De plus, les électrodes et les parois du récipient sont parallèles au niveau de fluide N pour obtenir des effluves régulières.Les électrodes 37 et 38 sont raccordées, par des conducteurs 42-43 à un générateur de courant 7, à haute tension et basse fréquence, de type rotatif, dont l'excitation est commandée par des moyens de régulation 39 sous la dépendance d'un débitmètre 40. Ainsi, en l'absence de toute circulation du fluide 31 dans le caisson, l'excitation du générateur est interrompue et les électrodes 37 et 38 ne sont pas alimentées. Par contre, dès que le fluide 31 circule dans le caisson, le générateur alimente les électrodes 37 et 38. Le champ électrique alternatif engendré entre ces électrodes génère, grâce aux parois diélectriques du caisson et à la couche d'air de la chambre 32, des effluves qui se répartissent à la surface du fluide et traversent ce dernier. Comme dans les autres formes d'exécution, ces effluves, d'une part, transforment l'oxygène de l'air en ozone et, d'autre part, détruisent les bactéries du fluide en mouvement, assurant ainsi une double action stérilisatrice.

Il faut noter que pour éviter tout arrêt de l'effluvage consécutif à la perte de conductibilité du gaz dans la chambre 32, provenant de la transformation de l'air en ozone, le robinet 35 est légèrement ouvert pour laisser échapper un débit de fuite, inférieur au débit d'admission de l'air, et permettant le renouvellement de l'air dans cette chambre 32.

Un tel dispositif peut être utilisé pour stériliser tout liquide conducteur véhiculé dans une conduite et par exemple, de l'eau pour l'alimentation humaine ou animale.

La figure 8 montre une variante de réalisation du dispositif de figure 6, variante dans laquelle l'électrode supérieure est constituée par un cylindre 50 qui, d'axe longitudinal transversal, c'est-à-dire perpendiculaire à la direction de déplacement des récipients 26, est composé d'un corps tubulaire 52 en matériau diélectrique, tel qu'en quartz, verre, silice ou porcelaine.Ce corps comporte intérieurement une couche 53 en matériau conducteur de l'électricité, tel qu'une peinture métallique, reliée électriquement par un circuit 54 à l'axe 55 du cylindre, lui-même relié par un circuit 56 au générateur 7.

Les génératrices du corps tubulaire 52 sont parallèles à la face supérieure de la bande 6a du transporteur, constituant l'autre électrode. La face extérieure du corps tubulaire 5 a un état de surface de qualité polie.

Enfin, le corps tubulaire est lié en rotation à l'arbre 55 lui-même relié à des moyens aptes à entraîner la rotation avec une vitesse circonférencielle sensiblement égale à la vitesse de défilement des récipients.

La figure 8 montre pour augmenter la puissance de traitement ou augmenter la vitesse de déplacement

5

des récipients, le cylindre 50 peut être associé à au moins un autre cylindre. Comme dans les formes d'exécution précédente le ou les cylindres sont en contact avec les récipients ou éloignés d'eux d'une distance comprise entre 0 et 5 mm.

En fonctionnement, un tel dispositif génère un flux d'effluves régulières et dense formant une nappe verticale transversale. Exceptée cette différence, le fonctionnement général du dispositif et les résultats obtenus sont les mêmes que ceux des formes d'exécution antérieures.

Ce dispositif peut être utilisé pour stériliser des produits alimentaires, des produits hygièniques, des produits médicaux et de manière général tout produit contenu dans un récipient en matéraiu diélectrique étanche aux bactéries. Il peut aussi être utilisé pour stériliser des sachets vides.

La figure 9 montre une forme d'exécution de l'application du dispositif à la stérilisation interne de sachets ou sacs, détachables d'une bande et délimités par des soudures 59 dans deux nappes supperposées 60-61 en un matériau diélectrique, monocouche ou multicouches, tel qu'en matière synthétique, papier ou complexes divers.

Il comprend une électrode cylindrique 50, de même structure que celle décrite dans la forme d'exécution précédente, et une électrode 62 en forme de gouttière coiffant la première sans la toucher. L'électrode 62 est réalisée en matériau conducteur de l'électricité, et par exemple en acier inoxydable ou en aluminium. Elle comporte une face active concave 63, dont l'état de surface est de qualité polie, c'est-à-dire a une rugosité R.A. de 0,4.

Le rayon intérieur R de la gouttière 62 est régulier et à une valeur supérieure à celle du rayon extérieur r du corps tubulaire de l'électrode 50.Le centre 01 de ce rayon R est décalé du centre 02 du rayon r d'une valeur S, inférieure à la valeur R-r, de manière que l'espace radial T, entre les deux électrodes, mesuré dans le plan médian P contenant l'axe longitudinal géométrique de l'électrode 52 et l'axe longitudinal géométrique de la gouttière, soit supérieur à l'épaisseur des nappes 60-61, et aille en croissant de part et d'autre de ce plan. En pratique, cet espace T a une valeur de l'ordre de 2 mm.

Les génératrices de l'électrode 62 sont parallèles à celles de l'électrode cylindrique afin que les distances radiales entre elles soient constantes sur leur longueur.

En fonctionnement, l'électrode 52 est entraînée en rotation dans le sens de la flèche 64 et entraîne avec elle la bande de récipients. Dès que celle-ci pénètre dans l'intervalle entre les deux électrodes, elle est soumise à un champ électrique transversal, traversant les nappes 60-61 la composant et formant, dans la couche d'air enfermée dans chacun des récipients de cette bande, des effluves électriques régulières, transformant l'oxygène de cet air en ozone et stérilisant l'intérieur de ces récipients. La densité des effluves et leur puissance de traitement, va en s'accroissant jusqu'à l'espace T pour décroître au delà, jusquà l'extrémité aval de l'électrode 62.

Les récipients ainsi obtenus sont stérilisés intérieurement et grâce à l'ozone qu'ils contiennent restent stériles jusqu'à leur ouverture. De tels récipients, peuvent recevoir, sans aucun risque de pollution, tous produits alimentaires ou médicaux, déjà stérilisés, ou devant subir ultérieurement un traitement de stérilisation moins poussé.

Pour faciliter la mise en place de la bande de récipient, mais aussi pour pouvoir s'effacer au passage d'une surépaisseur, l'électrode 62 est portée par un support, non représenté, apte à l'amener de sa position de travail, à une position escamotée, dans laquelle elle est très éloignée de l'électrode 50. Ce support est associé à des moyens coupant l'alimentation des deux électrodes dès que celle 62 n'occupe plus sa position de travail, afin d'éviter toute formation d'arcs électriques et éliminer tout danger pour le personnel.

Les bords amont 62a et aval 62b de l'électrode 62, comme d'ailleurs les bords latéraux et longitudinaux de toutes les électrodes statiques 5 sont arrondis pour supprimer toute amorçage d'arc par effet de pointe.

Les exemples ci-dessous illustrent la qualité de stérilisation obtenue avec les dispositifs selon l'invention.

Exemple 1 : Stérilisation de l'eau en flacon

Un flacon en verre, de type utilisé en laboratoire, ayant une contenance de 75 cl, une hauteur de 150 mm, une épaisseur de 2 mm et un diamètre de corps de 90 mm avec fermeture par bouchon conique en verre rodé, a été remplie d'eau courante. Ce flacon a été posé couché sur une électrode inférieure en aluminium ayant une épaisseur de 2 mm et a reçu contre sa partie supérieure une électrode en aluminium de 1 mm d'épaisseur, en forme de gouttière, correspondant à la forme du flacon, ayant 100 mm de long, 30 mm de large et des bords arrondis. Les faces des électrodes en contact avec le récipient avaient une rugosité R.A. de 0,4. La surface de contact entre le flacon et la bouteille était de sensiblement 3000 mm$^2$.

L'une des bornes du générateur a été raccordée à la terre et à l'électrode inférieure et l'autre borne à l'électrode supérieure. Le générateur a été réglé pour fournir à 3000 hertz, 19 Kilovolts et 20 milliampères pendant 15 secondes.

EP 0 287 480 B1

Exemple 2 : Stérilisation de serviettes, tissus et pansements adhésifs

Une serviette en papier de 120 × 120 cm, pliée en quatre, un morceau de tissu de mêmes dimensions est plié en quatre, de même qu'un pansement du commerce, sorti de son emballage, ont été emballés individuellement entre deux nappes en matière synthétique 0multicouches, soudées entre elles pour former des sachets carrés, de 80 mm de côté. Ces sachets ont été stérilisés par une installation du type représenté à la figure 8 comprenant une seule électrode cylindrique dont le corps tubulaire avait un diamètre extérieur de 90 mm et une épaisseur de 3,5 mm, et une électrode plane en aluminium de 2 mm. Le générateur fournissait un courant de 3000 hertz, à 12 Kilovolts et 15 milliampères. Le traitement a durée 15 secondes.

Exemple 3 : Stérilisation de l'intérieur de sachet

Les sachets ont été formés à partir d'une nappe en matière synthétique, multicouches, de 60 micromètres d'épaisseur, de 120 mm de large et 170 mm de long, repliée sur elle et soudée sur ces 3 autres bords pour former un sachet de 60 × 170 mm. Les sachets ont été traités par le dispositif décrit dans l'exemple 2, ci-dessus, pendant 15 secondes avec un réglage du générateur fournissant un courant de 3000 Hertz, 18 kilovolts et 20 milliampères.

Tous ces essais ont été effectués dans un local à 18°C ayant une hygrométrie comprise entre 40 et 50%.

Les flacons et sachets obtenus ont ensuite été envoyés pour analyse bactériologique consistant en une numération effectuée à 22°C après 72 heures et 37°C après 24 heures.

Pour effectuer l'analyse, chacun des sachets de l'exemple 3 ont reçu 3 ml d'eau stérilisé.

Les résultats d'analyse sont indiqués ci-dessous :

NUMERATION

| CONDITIONS D'ESSAIS | Eau de l'Ex 1 | Chacun des produits textiles de l'Ex 2 | sachets de l'Ex 3 | |
|---|---|---|---|---|
| | | | Traité | non traité |
| 22°C/72H | 0 germe dans 20 ml | 0 germe pour 4,6 grammes | 0 germe pour 1 ml | 133 germes pour 1 ml |
| 37°C/24H | 0 germe dans 20 ml | 0 germe pour 4,6 grammes | 0 germe pour 1 ml | 56 germes pour 1 ml |

Ce tableau montre clairement que le dispositif selon l'invention permet d'obtenir une stérilisation poussée avec des moyens simples, peu consommateur d'énergie, facilement adaptables en sortie de chaine de fabrication et sans danger pour le personnel.

**Revendications**

1. Procédé pour rendre stérile le contenu de récipients (3 ; 15 ; 19) en matériau diélectique étanche aux bactéries du type consistant à disposer les récipients entre des électrodes (5, 6) reliées aux bornes d'un générateur de courant électrique (7) à haute tension pour former entre elles un champ électrique alternatif engendrant, par effet CORONA, des effluves électriques, caractérisé en ce que, d'une part, chaque récipient est maintenu au contact de l'une des électrodes (6a) pendant la stérilisation, d'autre part, une meilleure répartition des effluves entre les électrodes (5, 6) est obtenue en masquant la partie métallique d'au moins l'une de ces électrodes (5) par une couche en matériau diélectrique (5a) et, de plus, les électrodes sont alimentées par un courant électrique alternatif de tension comprise entre 10000 et 50000 volts à une fréquence d'au moins 3000 Hertz.

7

2. Dispositif pour la mise en oeuvre du procédé selon la revendication 1 du type comprenant deux électrodes espacées (5, 6) reliées aux bornes d'un générateur de courant électrique (7) alternatif à haute tension et entre lesquelles chaque récipient (3 ; 15 ; 19) en matériau diélectrique est disposé pendant la stérilisation, caractérisé en ce que qu'au moins l'une des électrodes est composée d'une partie conductrice (5) à laquelle est associée, sur sa face faisant vis-à-vis à l'autre électrode (6a), une couche (5a) en matériau isolant constituant diélectrique d'égalisation et de répartition des effluves, tandis que, d'une part, au moins l'une de ces électrodes (6a) est en contact avec le récipient, que d'autre part, toutes les électrodes (5, 6) présentent des surface lisses, polies, sans aspérités et dont au moins les génératrices sont parallèles à celles de l'autre électrode et que de plus, les électrodes en vis-à-vis sont séparées par un intervalle (E) au plus égal à la dimension de la partie traitée du récipient augmentée d'une valeur comprise entre 0 et 5 mm et sont réliées à un générateur (7) apte à délivrer un courant alternatif de tension réglable comprise entre 10000 et 50000 volts à une fréquence d'au moins 3000 Hertz.

3. Dispositif selon la revendication 2, du type dans lequel les récipients sont déplacés par une bande transporteuse (6) caractérisé en ce que l'une des électrodes est constituée par la bande sans fin (6a), réalisée en matériau conducteur de l'électricité, tandis que l'autre électrode (5) est constitué par une plaque métallique qui, fixe et disposée parallèlement au dessus de la bande (6a), est solidaire d'une couche (5a) en matériau diélectrique faisant vis-à-vis à la bande (6a).

4. Dispositif selon la revendication 2, du type dans lequel les récipients sont déplacés par une bande transporteuse (6), caractérisé en ce que l'une des électrodes est constituée par la bande sans fin (6a), réalisé en matériau conducteur de l'électricité, tandis que l'autre électrode est constituée par un cylindre (50) qui, d'axe longitudinal parallèle au plan de déplacement de la bande (6a) et transversal à la direction de déplacement de celle-ci, est composé d'un corps tubulaire (52) en matériau diélectrique recouvert intérieurement par une couche (53), en matériau conducteur de l'électricité, reliée électriquement au générateur (7).

5. Dispositif selon la revendication 2 caractérisé en ce que dans le cas de son application au traitement de récipients formés par des nappes (60-61) supperposées, l'une des électrodes est constituée par un cylindre (50) qui, d'axe transversal à la direction de déplacement des récipients en nappes, est composé d'un corps tubulaire(52) en matériau diélectrique recouvert intérieurement par une couche (53) en matériau conducteur de l'électricité, reliée électriquement au générateur (7), tandis que l'autre électrode est constituée par une plaque métallique (62), en forme de gouttière, coiffant le cylindre précité, sans contact avec lui.

6. Dispositif selon la revendication 5 caractérisé en ce que l'électrode (62), en forme de gouttière, a un rayon intérieur (R) de valeur supérieure à la valeur du rayon extérieur (r) de l'electrode cylindrique (50) et de centre (01) décalé par rapport au centre (02) de cette électrode cylindrique, de manière que l'espace radial (T) entre les deux électrodes soit inférieur à leur différence de rayons et aille en croissant en s'éloignant de part et d'autre du plan médian (P) contenant leurs axes goémétriques longitudinaux.

7. Dispositif selon la revendication 2 caractérisé en ce que, dans le cas de son application à la stérilisation d'un fluide (31) apte à circuler dans un récipient (28) raccordé aux tronçons d'arrivée (29a) et de départ (29b) du fluide, le récipient est constitué par un caisson (28), en matériau diélectrique et étanche aux bactéries, ayant une hauteur (l) supérieure au diamètre intérieur (D) des tronçons et délimitant, entre sa paroi supérieure (28a) et le niveau supérieur du fluide qu'il contient, une chambre de traitement (32)contenant de l'air, tandis que, d'une part, les électrodes opposées (37-38), sont disposées à l'extérieur du caisson, respectivement contre sa paroi supérieure (28a) et contre sa paroi inférieure (28b), et que, d'autre part, débouchent dans la chambre de traitement des canalisations, respectivement, d'amenée (33) et d'évacuation (34) d'air sous pression, associées à des moyens (35-36) régulant le débit d'air sous pression pour maintenir sensiblement constant le niveau de fluide dans le caisson.

8. Dispositif selon la revendication 7 caractérisé en ce que les moyens de régulation du débit d'air sont constitués par un robinet (35) monté sur la canalisation (34) de sortie d'air et dont l'ouverture est sous la dépendance d'un détecteur (36) du niveau de fluide dans le récipient.

9. Dispositif selon l'ensemble des revendications 7 et 8 caractérisé en ce que l'excitation du générateur (7) est commandée par des moyens de régulation (39) sous la dépendance d'un débitmètre (40) mesurant le débit dans l'un des tronçons de conduite (29a-29b).

## Patentansprüche

1. Verfahren zur Sterilisierung des Inhalts von Behältern (3 ; 15 ; 19) aus dielektrischem bakteriendichtem Material, darin bestehend, daß die Behälter zwischen den an die Klemmen eines Hochspannungsgenerators (7) angeschlossenen Elektroden (5, 6) angeordnet sind, damit zwischen ihnen ein elektrisches Wechselfeld entsteht, so daß durch Koronaentladung elektrische Entladungen erzeugt werden, dadurch gekennzeichnet, daß einerseits jeder Behälter während der Sterilisierung in Berührung mit einer der Elektroden (6a) gehalten wird, daß andrerseits eine verbesserte Verteilung der Entladungen zwischen den Elektroden (5, 6) erreicht wird, indem das Metallstück zumindest einer dieser Elektroden (5) durch einen überzug aus dielektrischem Material (5a) verdeckt wird, wobei den Elektroden ein Wechselstrom zugeführt wird, dessen Spannung zwischen 10000 und 50000 Volt und dessen Frequenz zumindest 3000 Hertz beträgt.

2. Vorrichtung zur Durchführung des Verfahrens nach Anspruch 1, bei welcher zwei in Abstand stehende und an die Klemmen eines Hochspannungswechselstromsgenerators (7) angeschlossene Elektroden (5, 6) angebracht sind, zwischen denen jeder Behälter (3 ; 15 ; 19) aus dielektrischem Material während der Sterilisierung angeordnet wird, dadurch gekennzeichnet, daß mindestens eine der Elektroden aus einem stromführenden Teil (5) besteht, der auf der der anderen Elektrode (6a) zugewandten Seite mit einem überzug (5a) versehen ist, der aus einem Isolierstoff besteht, der als Dielektrikum wirkend die Entladungen ausgleicht und verteilt, während einerseits zumindest eine der Elektroden (6a) in Berührung mit dem Behälter steht, und andrerseits alle Elektroden (5, 6) glatte, polierte, unebenheitsfreie Oberflächen aufweisen und deren Generatrixen, wenigstens, mit denen der anderen Elektrode parallel verlaufen, und wobei die einander gegenüberliegenden Elektroden durch einen bestand (E) getrennt sind, der zumindest die um einen Wert von 0 bis 5 mm vermehrte Größe des behandelten Behälterteils beträgt, und an einen Generator (7) angeschlossen sind, der einen Wechselstrom, dessen Spannung zwischen 10000 und 50000 Volt einstellbar ist, mit einer Frequenz von mindestens 3000 Hertz zu liefern imstande ist.

3. Vorrichtung nach Anspruch 2, in der die Behälter auf einem Förderband (6) gefördert werden, dadurch gekennzeichnet, daß eine der Elektroden aus dem endlosen, aus stromführenden Material angefertigten Band (6a) besteht, während die andere Elektrode (5) aus einer Metallplatte besteht, die, unbeweglich und parallel über dem Band (6a) verlaufend angeordnet, mit einem dem Band (6a) gegenüberliegenden überzug (5a) aus dielektrischem Material haftend versehen ist.

4. Vorrichtung nach Anspruch 2, in der die Behälter auf einem Förderband (6) gefördert werden, dadurch gekennzeichnet, daß eine der Elektroden aus dem endlosen, aus stromführenden Material angefertigten Band (6a) besteht, während die andere Elektrode aus einer Walze (50) besteht, die eine der Bandbahn (6a) parallel verlaufende und der Verlaufrichtung des Bandes quertaufende Längsachse besitzt und aus einem röhrenförmigen Körper (52) aus dielektrischem Material besteht, das innerlich mit einem überzug (53) aus stromführendem Material versehen ist, der an den Generator (7) elektrisch an geschlossen ist.

5. Vorrichtung nach Anspruch 2, dadurch gekennzeichnet, daß im Falle seiner Anwendung auf die Behandlung von Behältern, die aus übereinanderliegenden Schichten (60-61) bestehen, eine der Elektroden aus einer Walze (50) besteht, die eine der Bewegungsrichtung der schichtenartigen Behälter querlaufende Achse besitzt und aus einem röhrenförmigen Körper (52) aus dielektrischem Material besteht, das innerlich mit einem überzug (53) aus stromführendem Material versehen ist, der an den Generator (7) elektrisch angeschlossen ist, während die andere Elektrode aus einer rinnenförmigen Metallplatte besteht, die die vorerwähnte Walze überdeckt, ohne sie zu berühren.

6. Vorrichtung nach Anspruch 5, dadurch gekennzeichnet, daß die rinnenförmige Elektrode (62) einen inneren Radius (R) aufweist, der einen größeren Wert hat, als den des äußeren Radius (r) der Walzenelektrode (50), und einen Mittelpunkt (01) besitzt, der im Vergleich zum Mittelpunkt (02) dieser Walzenelektrode verschoben ist, so daß der radiale Abstand (T) zwischen beiden Elektroden keiner ist, als ihr Radiusunterschied und immer mehr zunimmt, wenn die Entfernung auf der einen oder der anderen Seite der Mittelebene (P) wächst, in der ihre geometrischen Längsachsen stehen.

7. Vorrichtung nach Anspruch 2, dadurch gekennzeichnet, daß im Fall ihrer Anwendung auf die Sterilisierung einer Flüssigkeit (31), die dazu geeignet ist, in einem Behälter (28) zu fließen, der an die Endstücke für Zu- (29a) und Abfuhr (29b) der Flüssigkeit angeschlossen ist, der Behälter aus einem bakteriendichten, aus dielektrischem Material hergestellten Gefäß (28) besteht, dessen Anhöhe (I) größer ist als der innere Durchmesser (D) der Endstücke und der zwischen seiner oberen Wand (28a) und dem höchsten Stand der enthaltenen Müssigkeit eine luftenthaltende Behandlungskammer (32) abgrenzt, während einerseits die gegenüberliegenden Elektroden (37-38) außerhalb des Gefäßes angebracht sind, und zwar jeweils an dessen oberer Wand (28a) und an dessen unterer Wand (28b), und während andrerseits in die Behandlungskammer jeweils eine Druckluftzufuhrleitung (33) und eine Druckluftabfuhrleitung (34) einmünden, die mit Steuerungsmitteln (35-36) verkoppelt sind, die die Druckluftmenge regulieren, damit der Flüssigkeitsstand im Gefäß bedeutend gleichmäßig gehalten wird.

8. Vorrichtung nach Anspruch 7, dadurch gekennzeichnet, daß die Reguliermittel der Luftmenge aus einem an der Luftabfuhrleitung (34) angebrachten Drosselventil (35) besteht, dessen Steuerung von einem Fühler (36) des Flüssigkeitsstands im Behälter abhängig ist.

9. Vorrichtung nach beiden Ansprüchen 7 und 8, dadurch gekennzeichnet, daß die Erregung durch den Generator (7) von Regulierungsmitteln (39) gesteuert wind, die vom einem Durchflußmesser (40) abhängig sind, der die Durchflußmenge in einem der Leitungsabschnitte (29a-29b) abmißt.

## Claims

1. A process for sterilizing the content of bacteria-tight containers (3, 18, 19) made of a dielectric material, said process comprising placing the containers between electrodes (5-6), connected to the terminals of a high voltage electric current generator (7) to form an alternating electrical field therebetween generating electric CORONA discharges, characterized in that each container is kept in contact with one of the electrodes (6a) during the sterilization, and the distribution of the corona discharges between the electrodes (5, 6) is obtained by making the metal portion of at least one of the electrodes (5) with a layer made of dielectric material (5a) and, furthermore, the electrodes are supplied with an alternating electric current having a voltage of between 10,000 and 50,000 volts and a frequency of at least 3000 Hertz.

2. A device for using the process according to claim 1, said device comprising two spaced out electrodes (5, 6) connected to the terminals of a high voltage alternating electric current generator (7) and therebetween each container (3, 15, 19) made of a dielectric material is placed during the sterilization, characterized in that at least one of the electrodes is made up of a conductive part (5) with which is associated, on its face opposite to the other electrode (6a), a layer (5a) of insulating material constituting a dielectric for equalizing and distributing the discharges, with at least one of these electrodes (6a) being in contact with the container and, all the electrodes (5, 6) having smooth, polished surfaces, without roughnesses, of which at least the generatrix are parallel to those of the other electrode, and, furthermore, the opposing electrodes are separated by an interval (E) at most equal to the dimension of the treated part of the container increased by a value of between 0 and 5 mm and are connected to a generator (7) capable of delivering an alternating current of adjustable voltage between 10,000 and 50,000 volts at a frequency of at least 3000 Hertz.

3. A device, according to claim 2, of the type in which the containers are moved by a conveyor belt (6) characterized in that one of the electrodes consists of the endless belt (6a), made of an electrically conductive material, while the other electrode (5) consists of a metal plate which, stationary and placed in parallel above the conveying belt (6a), is solidly connected with a layer (5a) of dielectric material opposite to the belt (6a).

4. A device according to claim 2, of the type in which the containers are moved by a conveyor belt (6), characterized in that one of the electrodes consists of the endless belt (6a), made of an electrically conductive material, while the other electrode consists of a cylinder (50) which, with a longitudinal shaft parallel to the plane of movement of the belt (6a) and transverse to the direction of movement of the latter, comprises a tubular body (52) made of a dielectric material covered on the inside by a layer (53), of an electrically conductive material, electrically connected to the generator (7).

5. A device according to claim 2, characterized in that, in the case of its application to the treatment of containers formed of superposed sheets (60-61), one of the electrodes consists of a cylinder (50) which, with a shaft transverse to the direction of movement of the containers in sheet form, is made up of a tubular body (52) made of a dielectric material covered on the inside by a layer (53) of an electrically conductive material, electrically connected to the generator (7), while the other electrode consists of a metal plate (62), in the shape of a gutter, covering said cylinder, without contacting it.

6. A device according to claim 5 characterized in that the electrode (62), in the shape of a gutter, has an inner radius (R) of a value greater than the value of the outer radius (r) of the cylindrical electrode (50) and has a centre (01) offset relative to the centre (02) of this cylindrical electrode, so that the radial space (T) between the two electrodes is less than their difference in radii and continues to increase when moving away from both sides of the median plane (P) their geometrical longitudinal axes.

7. A device according to claim 2 characterized in that, in the case of its application to the sterilization of a fluid (31), capable of flowing in a container (28) connected to the fluid intake sections (29a) and the fluid outlet section (29b), the container consists of a bacteria-tight box (28) of a dielectric material, having a height (I) greater than the inner diameter (D) of the sections and delimiting, between its upper wall (28a) and the upper level of the fluid which it contains, a treatment chamber (32) containing air while, on the one hand, the opposing electrodes (37-38) are placed outside the box, respectively against its upper wall (28a) and against its lower wall (28b) and, on the other hand, a compressed air intake (33) and an outlet duct (34), associated to means (35-36) regulating the compressed air flow to keep the fluid level approximately constant,in the treatment chamber.

8. A device according to claim 7, characterized in that the means for regulating the air flow consist of a cock (35) mounted on the air outlet duct (34), the opening of which is dependant on a detector (36) of the fluid level in the container.

9. A device according to both of claims 7 and 8, characterized in that the excitation of the generator (7) is controlled by regulating means (39) depending on a flowmeter (40) measuring the flow in one of the duct sec-

tions (29a-29b).

FIG.1

FIG.5

FIG.2

FIG.3

FIG.6

FIG.4

FIG. 7

FIG.8

FIG. 9